# EUROPEAN PATENT APPLICATION

(11) **EP 2 862 593 A1**
(43) Date of publication of application: **22.04.2015**
(21) Application number: 13804050.6
(22) Date of filing: 11.06.2013
(51) Int. Cl.: A61M 27/00, A61M 1/00, A61M 3/00

(54) **WASHING SOLUTION AND WASHING METHOD**

(30) Priority: 15.06.2012 JP 2012136271
(71) Applicant: Ohdaira, Takeshi, Fukuoka-shi, Fukuoka 812-0054 (JP); Kyocera Medical Corporation, Osaka 532-0003 (JP)
(72) Inventor: OHDAIRA, Takeshi, Fukuoka-shi, Fukuoka 812-0054 (JP)
(74) Representative: Von Kreisler Selting Werner - Partnerschaft von Patentanwälten und Rechtsanwälten mbB
(86) International application number: PCT/JP2013/066077
(87) International publication number: WO 2013/187407

(57) **Abstract**

Provided is a technique for washing a body fluid, a foreign body or excrement in a living body rapidly and cleanly without damaging biological tissues. A body fluid, a foreign body or excrement in a living body can be washed by bringing the body fluid, the foreign body or the excrement into contact with a washing solution containing micro-bubble water.

## Description

### TECHNICAL FIELD

The present invention relates to a washing solution used for washing a body fluid, a foreign body, excrement, and the like in a living body, and a method for washing a body fluid, a foreign body, excrement, and the like in a living body.

### BACKGROUND ART

Recently, a study on washing by liquid including micro-bubbles is being carried out, and thus, the various washing methods for various objects are reported.

For example, Patent Document 1 discloses a method of using nano-bubbles and an apparatus for generating the nano-bubbles. Patent Document 1 discloses a method of using nano-bubbles by using characteristics, such as, sterilizing action, and surfactant action by the decrease in buoyancy caused by nano-bubbles, the increase in surface area, the increase in surface activity, the formation of local high-pressure field, and the realization of electrostatic polarization.

In detail, by relating these characteristics and actions to each other, the nano-bubbles exhibit an absorbing effect of dust ingredients (a detaching effect of dust ingredients), a high-speed washing effect on the surface of object, and a sterilizing effect, for various objects. In this way, various objects can be washed with low environmental load, thereby purifying contaminated water. In addition, it is described that the nano-bubbles can be applied into a living body, and thus, can be used for fatigue recovery.

Patent Document 2 discloses the technique of an anus washing apparatus including a nano-bubbles generator that generates nano-bubbles in liquid and a nozzle that spouts a fluid including the nano-bubbles generated by the nano-bubbles generator. It is described that since the nano-bubbles have an ultrafine form, when an anus is washed with the liquid including the nano-bubbles, the nano-bubbles move in an anus and permeate a rectum region, and thereby, the sterilizing action and washing action in those regions are performed.

Patent Document 3 discloses the technique for installing, at the upper part of a pressure-adjusting tank, a micro-bubbles-generating nozzle for collecting the bubbles with large size in the upper space of the inside of the tank by installing the pressure-adjusting tank in a circuit in a micro-bubbles generator, and thus, float-separating the bubbles with large size, and also, for restoring gas as the micro-bubbles in liquid by aspirating the gas from the upper space thereof.

### CITATION LIST

### PATENT DOCUMENT

Patent Document 1: JP 2004-121962 A
Patent Document 2: JP 2008-291521 A
Patent Document 3: JP 2011-206689 A

### SUMMARY OF THE INVENTION

### PROBLEM TO BE SOLVED BY THE INVENTION

When a body fluid or a foreign body in a living body, which is generated in a affected area and the surrounding area of the affected area for an operation or treatment, should be removed, it is preferable to remove rapidly and cleanly them.

By the way, Patent Document 1 discloses washing the living bodies, such as, perishable foodstuff and Patent Document 2 discloses washing an anus. However, they do not disclose the applications of the washing technique using the liquid including micro-bubbles for removing a body fluid or a foreign body in a living body.

The reason is that a high degree of effort is variously required, such that the extremely sensitive biological tissues are not damaged. In detail, the liquid (washing solution) capable of washing rapidly and cleanly by making the liquid without damaging the biological tissues and also by increasing the washing effect is required.

This object is widely present in general surgical operations accompanying bleeding, which require washing the inside of skull, the insides of the abdominal cavity and body cavity, and the inside of intestinal tract.

Therefore, an object of the present invention is to provide the technique for washing rapidly and cleanly a body fluid, a foreign body, and excrement in a living body, without damaging the biological tissues.

### MEANS FOR SOLVING PROBLEM

The present inventors studied for finding the technique of washing rapidly and cleanly a body fluid, a foreign body, and excrement in a living body, without damaging the biological tissues, and as a result, found that the body tissues can be rapidly and cleanly washed using the washing solution including micro-bubble water without damaging the body tissues. Therefore, the present invention completed.

For achieving the above object, the present invention is to provide a washing solution for washing a body fluid, a foreign body, and excrement in a living body, which includes micro-bubble water obtained by mixing gas and liquid.

By using the washing solution including the micro-bubble water, a body fluid, a foreign body, and excrement in a living body can be rapidly and cleanly washed without damaging the biological tissues.

The washing solution is preferable for washing a body fluid having blood as a main component. Especially, it is preferable for washing blood clots.

The washing solution is preferable for washing blood generated in the surrounding area of the affected area at the time of operating or treating.

The washing solution is particularly preferable for isolating jelly hematoma adhering to the intracranial tissues from the intracranial tissues and for disintegrating the jelly hematoma to be non-jelly form.

The washing solution is particularly preferable for isolating the excrement adhering to the inside of intestinal tract.

It is preferable that the micro-bubble in the micro-bubble water have the median diameter of 30 to 60 µm and the particle diameter range of 5 to 200 µm.

By using the washing solution including the micro-bubble water having such particle size distribution, a body fluid, a foreign body, and excrement in a living body can be very effectively washed without damaging a living body.

In addition, for achieving the above object, the present invention is to provide a method for washing a body fluid, a foreign body, or excrement in a living body, which includes bringing a washing solution including micro-bubble water into contact with the inside of the living body.

The method may include washing a body fluid including blood as a main component.

The method may include washing blood generated in the surrounding area of the affected area at the time of operating or treating.

The method may include isolating jelly hematoma adhering to the intracranial tissues from the intracranial tissues and disintegrating the jelly hematoma to be non-jelly form.

The method may include isolating excrements adhering to the intestinal tract.

The median diameter of micro-bubble in the micro-bubble water used for the above-described method is preferably 30 to 60 µm. In addition, preferably, the particle size diameters of all the bubbles are substantially in the range of 5 to 200 µm.

By using the washing solution including the micro-bubble water having such particle size distribution, a body fluid, a foreign body, and excrement in a living body can be very effectively washed without damaging a living body.

### EFFECT OF THE INVENTION

According to the present invention, it is possible to wash rapidly a body fluid, a foreign body, and excrement in a living body without damaging biological tissues.

In detail, according to the present invention, it is possible to wash effectively and safely the inside of skull, the insides of the abdominal cavity and body cavity, and the inside of intestinal tract for surgical operations.

### BRIEF DESCRIPTION OF DRAWINGS

Fig. 1 is a diagram illustrating a particle size distribution of micro-bubble physiological saline produced according to Example;
Fig. 2 is a photograph illustrating a result of washing a blood clot region using micro-bubble physiological saline; and
Fig. 3 is a photograph illustrating a result of washing a blood clot region using general physiological saline.

### MODE(S) FOR CARRYING OUT THE INVENTION

In the present invention, "micro-bubble water" is defined as the liquid including micro-bubbles that have substantially a particle size distribution of micro order (for example, 95% or more of particles). In other words, such concept is different from the micro-bubble water having the micro-bubbles of a micro order (several nm to several hundred nm) as a main constituent.

A preferred particle size distribution of the micro-bubble water used for the present invention will be described.

Median diameter (50% particle diameter): preferably 30 to 60 µm, more preferably 35 to 55 µm, and still more preferably 40 to 50 µm

90% particle diameter: preferably 50 to 80 µm, more preferably 55 to 75 µm, and still more preferably 60 to 70 µm

10% particle diameter: preferably 10 to 40 µm, more preferably 15 to 35 µm, and still more preferably 20 to 30 µm

In addition, preferably, the particle diameters of all the bubbles in the micro-bubble water used for the present invention are substantially in the range of 5 to 200 µm.

For the present invention, the particle diameter described above is measured using the measurement principle of a laser diffraction method. As a laser diffraction particle size analyzer, a laser diffraction particle size analyzer HELOS & RODOS manufactured by Sympatec GmbH may be used.

By using the washing solution including micro-bubble water having the particle size distribution described above, the body fluids including proteins, and the like can be effectively washed.

The washing solution of the present invention may include the same components as the general washing solution capable of being used for washing the inside of living body, except that it has micro-bubbles. The washing solution of the present invention is preferably composed of micro-bubble physiological saline.

The washing solution of the present invention can be produced using a micro-bubble generator including a pump that mixes gas and water, and an orifice nozzle that makes the gas included in the mixed water thus obtained to be micro-bubbles.

In more detail, it is possible to adjust a particle diameter by adjusting the diameter of the orifice-type nozzle in the micro-bubble generator.

In addition, by passing through a plurality of orifice-type nozzles, it is possible to adjust a particle size distribution.

In addition, the bubble water produced in the micro-bubble generator is stored in a hanging-type vessel and is allowed to be spontaneously dropped by self-weight, and thereby, the micro-bubble water can be sent to a washing tube at a low flow rate and low pressure.

In the preferred embodiment of the present invention, the hanging-type vessel is provided at the upward position as compared with the object to be washed (washing position in the inside of living body), such that the washing tube for a spontaneous dropping of the micro-bubble water is extended from the hanging-type vessel.

Therefore, when the washing tube for a spontaneous dropping of the micro-bubble water is constituted to be extended from the hanging-type vessel that is provided at the upward position as compared with the object to be washed, it is possible to adjust the discharging pressure of the micro-bubble water by changing only a height of the vessel position. In this way, it is possible to make the discharging pressure of the micro-bubble water to be the discharging pressure that is suitable for the object to be washed.

### Example

Micro-bubble water was produced using the micro-bubble generator as described above with physiological saline.

The particle size distribution of the micro-bubble water thus produced was measured by the following method.

The micro-bubble water (white turbidity) produced from the micro-bubble generator was supplied in a 250 ml beaker for 1 minute.

Next, the micro-bubble water was transferred into a measuring vessel (50 ml volume), and then, after 15 seconds, the measurement was started. It was measured 50 times (for 5 seconds) at 0.1 second/one time as a measuring time. The measurement was performed using a laser diffraction particle size analyzer HELOS & RODOS manufactured by Sympatec GmbH.

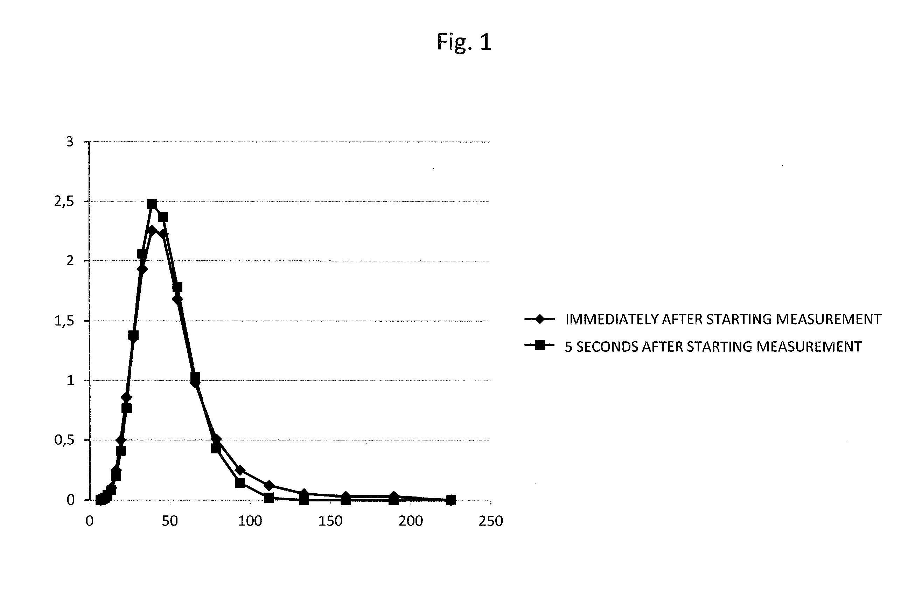

The results thus measured are illustrated in Fig. 1.

As the measuring results, the bubbles of the micro-bubble water had the following particle diameters.
Median diameter (50% particle diameter): 42 µm
90% particle diameter: 62 µm
10% particle diameter: 22 µm

In addition, the particle diameters of all the bubbles in the micro-bubble water were substantially in the range of 5 to 200 µm.

The following washing test was performed using the physiological saline produced as described above.

The subcutaneous inside diameter artery of the pig was exposed and a part thereof was incised, thereby causing a bleeding. Since then, the blood clots that were formed around the incision site in a few minutes were washed with physiological saline and micro-bubble physiological saline.

In detail, a washing tube was connected to the micro-bubble generator, and then, the inside of the pig body was washed with the produced micro-bubble physiological saline. In addition, as Comparative Example, it was washed with physiological saline.

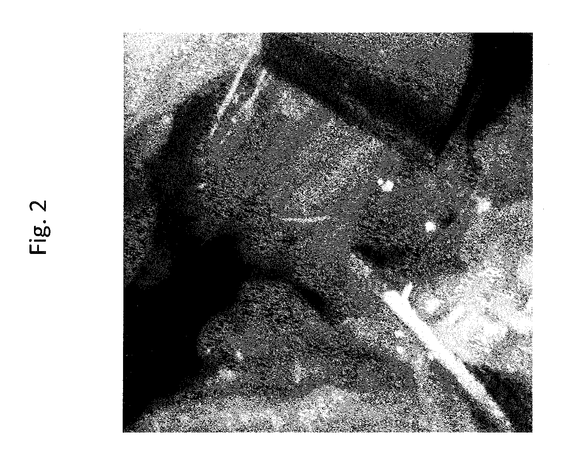

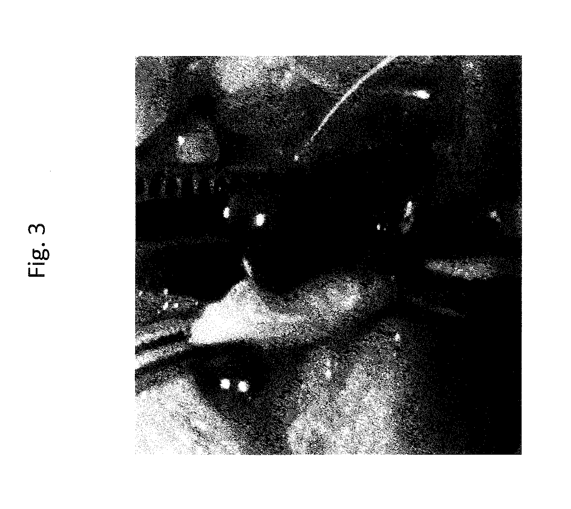

The results thus obtained are illustrated in Figs. 2 and 3. Fig. 2 illustrates a result of washing with the micro-bubble physiological saline, and Fig. 3 illustrates a result of washing with the physiological saline.

In the case of using the physiological saline, the blood clots were not disintegrated by washing, and thus, remained as a jelly form. When the blood clots were not removed, it was difficult to secure the view of the operation region. In other words, the effect of the physiological saline was not high.

Meanwhile, the blood clots were disintegrated by washing in the case of using the micro-bubble physiological saline. Therefore, as compared with the case of using the physiological saline, the view of the operation region was secured. In other words, it could be confirmed that the washing effect of the micro-bubble physiological saline was very high.

Generally, since it begins to form the blood clots in several minutes, the coagulation of blood is inevitable for a general operation. Therefore, it was confirmed that the micro-bubble physiological saline was very useful even in terms of the security of view in the operation regions.

From the results of Example, it can be expected that the blood clots in the inside of skull, such as epidural hematoma may be very effectively washed.

In addition, it can be expected that the inside of intestinal tract that is characterized in prolonged bleeding and is attached with pus, necrotic tissues, and excrements may be very effectively washed.

In addition, it is reported that micro-bubble water generates the bubbles having a nano-bubble size of 0.05 µm to 0.1 µm during a squeezing process. The washing solution including the micro-bubble water according to the present invention may include such a nano-bubble. It can be expected that the washing solution has the above-described washing effect as well as a sterilizing effect.

### INDUSTRIAL APPLICABILITY

The present invention can be used for washing a body fluid, a foreign body, and excrement in a living body.

## Claims

1. A washing solution comprising micro-bubble water obtained by mixing gas and liquid for use in washing a body fluid, pus, a necrotic tissue, or excrement in a living body.

2. The washing solution according to claim 1, wherein the body fluid has blood as a main component.

3. The washing solution according to claim 2, wherein the blood is generated in a surrounding area of affected area at the time of operating or treating.

4. The washing solution according to claim 1, for use in isolating jelly hematoma adhering to an intracranial tissue from the intracranial tissue and disintegrating the jelly hematoma to be non-jelly form.

5. The washing solution according to claim 1, for use in isolating and washing the excrement adhering to an intestinal tract.

6. The washing solution according to any one of claims 1 to 5, wherein a median diameter of bubbles in the micro-bubble water is 30 to 60 µm.

7. The washing solution according to any one of claims 1 to 6, wherein a particle diameter of the bubbles in the micro-bubble water is in the range of 5 to 200 µm.

8. The washing solution according to any one of claims 1 to 7, wherein the micro-bubble water further comprises nano-bubbles in the range of particle diameter of 0.05 µm to 0.1 µm.

9. A washing method for washing a body fluid, a foreign body, and excrement in a living body, comprising bringing a washing solution including micro-bubble water obtained by mixing gas and liquid into contact with the inside of a living body.

10. The washing method according to claim 9, wherein the body fluid has blood as a main component.

11. The washing method according to claim 10, wherein the blood is generated in a surrounding area of affected area at the time of operating or treating.

12. The washing method according to claim 9, comprising isolating jelly hematoma adhering to an intracranial tissue from the intracranial tissue and disintegrating the jelly hematoma to be non-jelly form.

13. The washing method according to claim 9, comprising isolating excrement adhering to an intestinal tract.

14. The washing method according to any one of claims 9 to 13, wherein a median diameter of bubbles in the micro-bubble water is 30 to 60 µm.

15. The washing method according to any one of claims 9 to 14, wherein a particle diameter of the bubbles in the micro-bubble water is in the range of 5 to 200 µm.

16. The washing method according to any one of claims 9 to 15, wherein the micro-bubble water further comprises nano-bubbles in the range of a particle diameter of 0.05 µm to 0.1 µm.
